# EUROPEAN PATENT APPLICATION

(11) **EP 1 595 501 A1**
(43) Date of publication of application: **16.11.2005**
(21) Application number: 04712201.5
(22) Date of filing: 18.02.2004
(51) Int. Cl.: A61B 8/00

(54) **ULTRASONOGRAPHIC DEVICE**

(30) Priority: 18.02.2003 JP 2003040175
(71) Applicant: MATSUSHITA ELECTRIC INDUSTRIAL CO., LTD., Kadoma-shi, Osaka 571-8501 (JP)
(72) Inventor: NISHIGAKI, Morio, - (JP)
(74) Representative: Diehl, Hermann O. Th.
(86) International application number: PCT/JP2004/001843
(87) International publication number: WO 2004/073520

(57) **Abstract**

An ultrasonic diagnostic apparatus is provided that can achieve an excellent image quality even in the case where a subject moves at a high speed. A memory (3) stores a first signal obtained through first-time transmission/reception. When a second signal is obtained through second-time transmission/reception, a delay amount calculator (6) calculates a delay amount for either of the first and second signals based on first and second timings outputted respectively from first and second zero crossing detectors (4, 5), at which the first and second signals cross zero, respectively. Delayers (7, 8) delay the first or second signal by the calculated delay amount so that phases of the signals are matched with each other. An adder (9) adds together the two signals whose phases have been matched.

## Description

### Technical Field

The present invention relates to an ultrasonic diagnostic apparatus that makes an observation and a diagnosis of the state of a subject such as a living body or the like by performing synthetic aperture scanning in which a plurality of times of transmission/reception of ultrasonic beams is performed by arrayed transducer-elements.

### Background Art

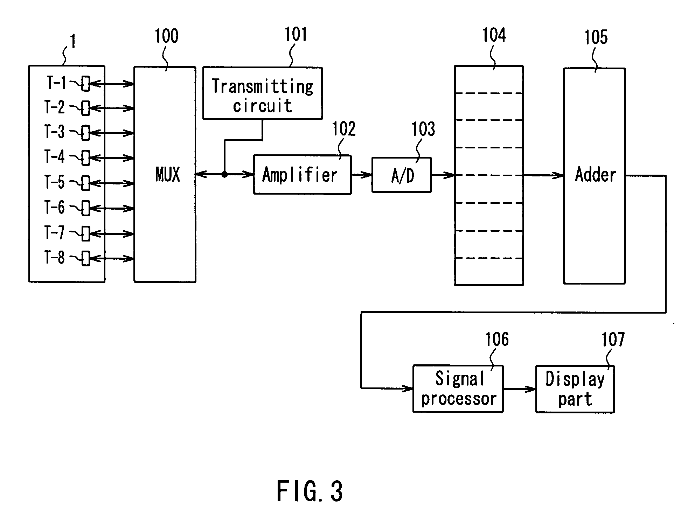

In recent years, an ultrasonic diagnostic apparatus that performs synthetic aperture scanning has been known. Such an ultrasonic diagnostic apparatus has been introduced in P. D. Corl, et al. "A digital synthetic imaging system for NDE", Proc IEEE Ultrasonics Symp., Sept. 1978. The following description is directed to the principle of the operation of the ultrasonic diagnostic apparatus with reference to FIG. 3. FIG. 3 is a block diagram schematically showing an example of the configuration of an ultrasonic diagnostic apparatus using synthetic aperture scanning in Conventional Example 1.

In FIG. 3, an ultrasonic probe 1 is composed of a plurality of transducer-elements T1 to T8. The transducer-element Tn (n = 1 to 8) generates ultrasonic pulses, and the ultrasonic pulses reflected in a subject are received by the transducer-element Tn as an echo ultrasonic wave. A received signal received by the transducer-element Tn passes through a multiplexer (MUX) 100 and is amplified by an amplifier 102. The received signal then is converted into digital data by an A/D 103 and written into a memory 104. Upon completion of the writing of the received signal from the transducer-element Tn into the memory 104, the MUX 100 subsequently selects the transducer-element Tn' that is different from the transducer-element Tn, and in the same manner as in the case of the transducer-element Tn, a received signal is written into the memory 104. In the above-described manner, received signals obtained by the transducer-elements T1 to T8 are written into the memory 104. Next, in an adder 105, output signals from the memory 104, namely, the received signals obtained by the transducer-elements T1 to T8 that have been stored in the memory 104, are added together after being provided with respective predetermined time differences.

Transmission/reception sequences (the above-described series of transmission/reception operations are referred to as a transmission/reception sequence) by the transducer-elements T1 to T8 are performed one by one in the above-described manner, and signals are integrated. Thus, the same effect as in the case of simultaneous reception by the eight transducer-elements can be obtained.

Assuming that the subject stays at rest during the periods of reception by the transducer-elements T1 to T8, in the subject, receiving directivity for beam convergence, beam deflection or the like can be imparted to the ultrasonic probe 1.

The received signals added by the adder 105 as described above are subjected to signal processing such as detection or the like by a signal processor 106 and displayed on a display part 107.

However, the above-described conventional ultrasonic diagnostic apparatus having a synthetic aperture has presented a problem that a synthetic aperture cannot be operated accurately in the case where a subject has moved during the periods of reception by the transducer-elements T1 to T8.

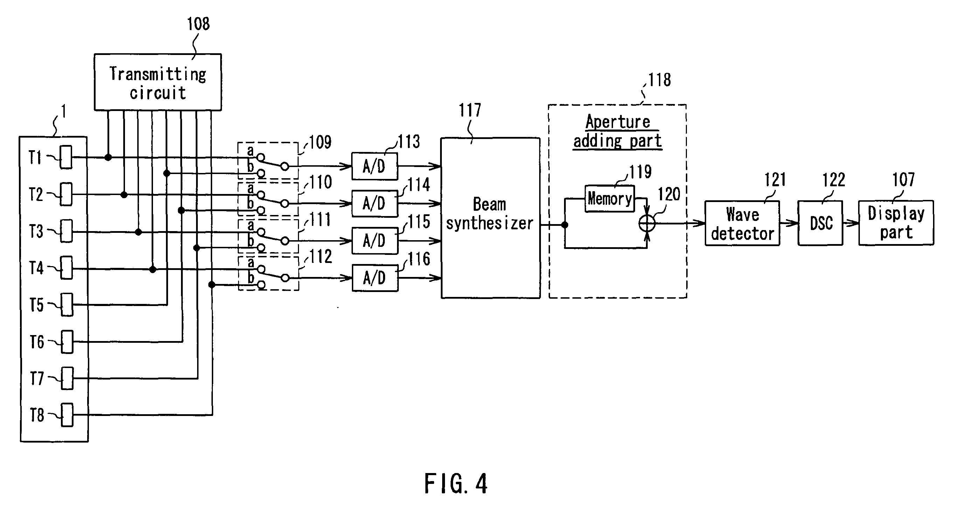

By referring to FIG. 4, the description is directed next to a method for improving a synthetic aperture in the case where a subject moves. FIG. 4 is a block diagram schematically showing an example of the configuration of the ultrasonic diagnostic apparatus using the synthetic aperture scanning as Conventional Example 2.

In FIG. 4, a probe 1 is composed of transducer-elements T1 to T8. The probe 1 is driven with high-voltage pulses generated by a transmitting circuit 108 and irradiates ultrasonic waves into a body that is not shown. Ultrasonic signals reflected in the body are received by the transducer-elements T1 to T8 and converted into electric signals. This transmission/reception sequence is performed twice. In first-time transmission/reception, switches 109 to 112 are connected with a-side contacts and select received signals from the transducer-elements T1 to T4, respectively. The signals that have passed though the SW 109 to 112 are converted into digital signals by A/D converters 113 to 116, respectively, and a beam is synthesized by a beam synthesizer 117. A signal obtained in the first-time transmission/reception is stored in a memory 119 in an aperture adding part 118.

Subsequently, second-time transmission/reception is performed. In this case, the SW 109 to 112 are switched so as to be connected with b-side contacts, and the transducer-elements T5 to T8 are selected. In the same manner as in the case of the first-time transmission/reception, received signals from the transducer-elements T5 to T8 are converted into digital signals by the A/D converters 113 to 116, respectively, and beam synthesizing is performed by the beam synthesizer 117. By an adder 120, a received signal obtained through the second-time transmission/reception is added to the signal obtained in the first-time transmission/reception that has been stored in the memory 119. After that, the signals are detected by a wave detector 121, scanned and converted by a digital scan converter (DSC) 122, and displayed on a display part 107.

As described above, in the method employed in the case of Conventional Example 2, the transmission/reception sequences for a synthetic aperture consist of two times of transmission/reception, and therefore, compared with the case of the synthetic aperture of Conventional Example 1, less influence is exerted by the movement of a subject.

However, the method employed in the case of Conventional Example 2 also presents the following problem. That is, according to the speed of the movement of a subject, a phase difference is caused between a signal obtained through first-time transmission/reception and a signal obtained through second-time transmission/reception, and the signals cancel each other when added by the adder 120. In the case where the cancellation is of such a high degree as to affect an image, the image quality might be deteriorated by the influence of the movement.

### Disclosure of Invention

With the foregoing in mind, it is an object of the present invention to provide an ultrasonic diagnostic apparatus that can achieve an excellent image quality even in the case where a subject moves at a high speed. That is, the present invention is to obtain an ultrasonic diagnostic apparatus that eliminates a phase difference between signals caused due to the movement of a subject during a plurality of times of transmission/reception by a synthetic aperture, thereby achieving an excellent image quality.

In order to achieve the above-mentioned object, a first ultrasonic diagnostic apparatus according to the present invention is an ultrasonic diagnostic apparatus using synthetic aperture scanning in which one beam is synthesized through a plurality of times of transmission/reception and includes: a transmitting circuit that transmits driving pulses a plurality of times; a plurality of arrayed transducer-elements, each of which emits an ultrasonic beam from an aperture according to the driving pulses, receives the ultrasonic beam reflected in a subject by means of the aperture, and outputs a received signal; switches that selectively output a plurality of signals from among the received signals outputted from the arrayed transducer-elements; a beam synthesizer that performs beam formation based on the signals selected by the switches; a memory that temporarily stores each of signals outputted from the beam synthesizer as a result of the plurality of times of transmission/reception; and an adder that adds together the signals in the memory that correspond respectively to the plurality of times of transmission/reception. The ultrasonic diagnostic apparatus further includes: a unit for detecting a phase difference between the signals obtained through the plurality of times of transmission/reception; and a delay unit that outputs to the adder the signals obtained through the plurality of times of transmission/reception while matching phases of the signals with each other. In this case, as the plurality of times of transmission/reception, for example, two times of transmission/reception are performed.

According to this configuration, a phase difference between a received signal obtained through first-time transmission/reception and a received signal obtained through second-time transmission/reception is eliminated, and thus the deterioration of an image quality can be prevented.

Preferably, in the first ultrasonic diagnostic apparatus, the unit for detecting a phase difference includes: a plurality of zero crossing detectors, each of which detects a timing at which an amplitude of each of the signals that correspond respectively to the plurality of times of transmission/reception shifts from a positive polarity to a negative polarity or vice versa to cross zero; and a unit for calculating a delay amount with respect to the signals obtained through the plurality of times of transmission/reception based on the timing detected by each of the plurality of zero crossing detectors.

This enables easy correction of a phase difference between signals obtained through a plurality of times of transmission/reception.

In order to achieve the above-mentioned object, a second ultrasonic diagnostic apparatus according to the present invention is an ultrasonic diagnostic apparatus using synthetic aperture scanning in which one beam is synthesized through a plurality of times of transmission/reception and includes: a transmitting circuit that transmits driving pulses a plurality of times; a plurality of arrayed transducer-elements, each of which emits an ultrasonic beam from an aperture according to the driving pulses, receives the ultrasonic beam reflected in a subject by means of the aperture, and outputs a received signal; switches that selectively output a plurality of signals from among the received signals outputted from the arrayed transducer-elements; a beam synthesizer that performs beam formation based on the signals selected by the switches; a memory that temporarily stores each of signals outputted from the beam synthesizer as a result of the plurality of times of transmission/reception; and an adder that adds together the signals in the memory that correspond respectively to the plurality of times of transmission/reception. The ultrasonic diagnostic apparatus further includes a plurality of wave detectors that perform amplitude detection of the signals obtained through the plurality of times of transmission/reception and output the signals to the adder. In this case, as the plurality of times of transmission/reception, for example, two times of transmission/reception are performed.

According to this configuration, canceling-out of signals caused due to a phase difference is prevented, and thus the deterioration of an image quality can be prevented.

### Brief Description of Drawings

FIG. 1 is a block diagram schematically showing an example of the configuration of an ultrasonic diagnostic apparatus using synthetic aperture scanning according to Embodiment 1 of the present invention.
FIG. 2 is a block diagram schematically showing an example of the configuration of an ultrasonic diagnostic apparatus using synthetic aperture scanning according to Embodiment 2 of the present invention.
FIG. 3 is a block diagram schematically showing an example of the configuration of an ultrasonic diagnostic apparatus using synthetic aperture scanning as Conventional Example 1.
FIG. 4 is a block diagram schematically showing an example of the configuration of the ultrasonic diagnostic apparatus using the synthetic aperture scanning as Conventional Example 2.

### Description of the Invention

Hereinafter, the present invention will be described by way of preferred embodiments with reference to the appended drawings.

### (Embodiment 1)

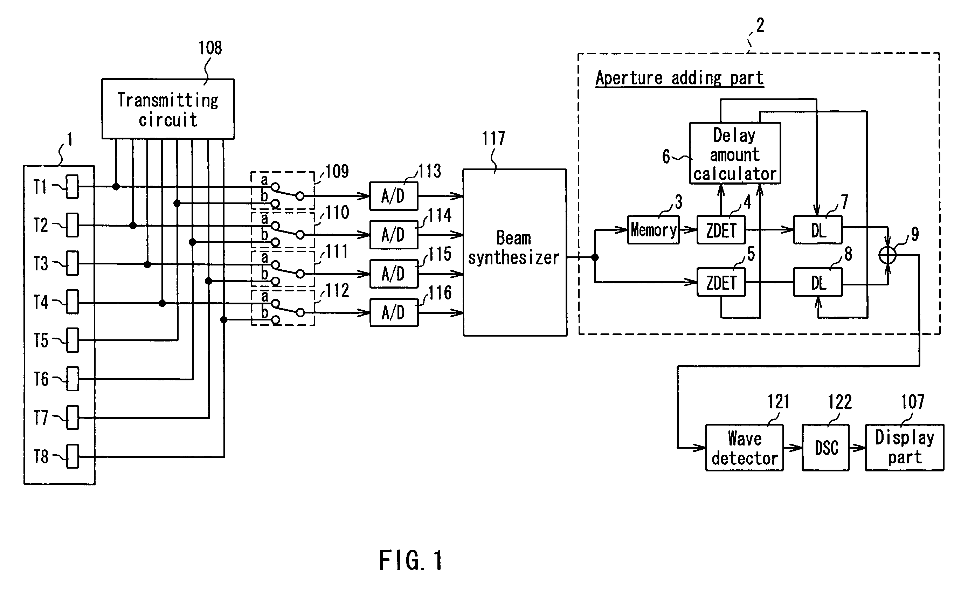

FIG. 1 is a block diagram schematically showing an example of the configuration of an ultrasonic diagnostic apparatus using synthetic aperture scanning according to Embodiment 1 of the present invention. In FIG. 1, the same reference numerals indicate the respective parts having similar configurations and functions to those in FIG. 4 referred to for describing Conventional Example 2 so as to avoid duplicate descriptions thereof.

In FIG. 1, in the same manner as in the case of Conventional Example 2, using a probe 1, signals for a synthetic aperture are received through two times of transmission/reception, and beam synthesization is performed. This embodiment is different from Conventional Example 2 in the configuration of an aperture adding part.

An aperture adding part 2 is composed of: a memory 3 that stores a signal (first signal) obtained through first-time transmission/reception; a first zero crossing detector 4 that detects a first timing at which an amplitude of the first signal obtained through the first-time transmission/reception shifts from a positive polarity to a negative polarity or vice versa to cross zero; a second zero crossing detector 5 that detects a second timing at which an amplitude of a second signal obtained through second-time transmission/reception shifts from a positive polarity to a negative polarity or vice versa to cross zero; a delay amount calculator 6 that calculates, based on the first and second timings outputted from the two zero crossing detectors 4 and 5, a delay amount determining whether and how much the first signal or the second signal should be delayed in order that phases of the first signal and the second signal are matched with each other; delayers 7 and 8 for delaying the first signal or the second signal by the delay amount calculated by the delay amount calculator 6; and an adder 9 that adds together the two signals whose phases have been matched by delaying.

The above-described configuration operates as follows. Initially, first-time transmission/reception is performed, and a received signal (first signal) is accumulated in the memory 3. Subsequently, second-time transmission/reception is performed to obtain a received signal (second signal), and based on this received signal, the first timing at which the received signal shifts from a positive polarity to a negative polarity or vice versa is detected by the zero crossing detector 5. At the same time, the received signal obtained through the first-time transmission/reception is read out from the memory 3, and the second timing at which the received signal shifts from a positive polarity to a negative polarity or vice versa is detected by the zero crossing detector 4. Based on information of the first and second timings detected by the two zero crossing detectors 4 and 5, it is judged by the delay amount calculator 7 whether and how much the first signal or the second signal is advanced (delayed). Delay amounts for the delayers 7 and 8 are adjusted so that phases of the received signals are adjusted to be the same, and the received signals are added together by the adder 9.

As described above, according to this embodiment, even in the case where a subject moves at a high speed, a phase difference between a received signal obtained through first-time transmission/reception and a received signal obtained through second-time transmission/reception is eliminated, and thus the deterioration of an image quality can be prevented.

In this embodiment, the memory 3 is located upstream of the zero crossing detector 4. However, this order of arrangement may be reversed. In that case, when a received signal is captured in the memory 3 as a result of first-time transmission/reception, the first timing at which the received signal shifts from a positive polarity to a negative polarity or vice versa could be detected and stored in the delay amount calculator 7.

Furthermore, this embodiment uses the timing detection by the zero crossing detectors 4 and 5 in which a timing at which an amplitude of a signal shifts from a positive polarity to a negative polarity or vice versa to cross zero is detected because it enables easy phase detection. However, other methods such as, for example, a method using a frequency analysis also may be used.

### (Embodiment 2)

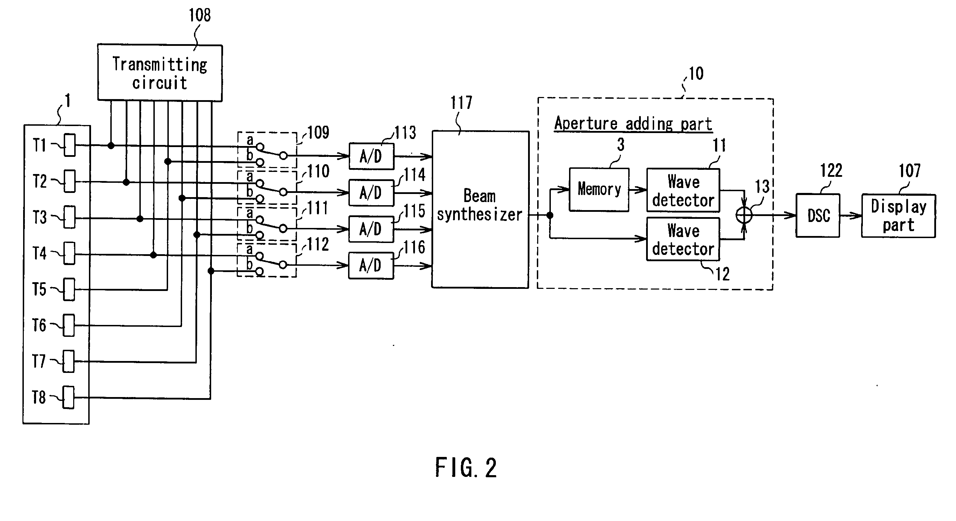

FIG. 2 is a block diagram schematically showing an example of the configuration of an ultrasonic diagnostic apparatus using synthetic aperture scanning according Embodiment 2 of the present invention. In FIG. 2, the same reference numerals indicate the respective parts having similar configurations and functions to those in FIGs. 4 and 1 referred to for describing Conventional Example 2 and Embodiment 1, respectively, so as to avoid duplicate descriptions thereof.

In FIG. 2, in the same manner as in the cases of Conventional Example 2 and Embodiment 1, using a probe 1, signals for a synthetic aperture are received through two times of transmission/reception, and beam synthesization is performed. This embodiment is different from Embodiment 1 in the configuration of an aperture adding part and does not require a wave detector 121.

In FIG. 2, an aperture adding part 10 is composed of a memory 3 that stores a received signal (first signal) obtained through first-time transmission/reception; a wave detector 11 that detects the received signal (first signal) obtained through the first-time transmission/reception; a wave detector 12 that detects a received signal (second signal) obtained through second-time transmission/reception; and an adder 13 that adds together the first and second signals that have been detected.

As described above, according to this embodiment, each of phase informations on a received signal obtained through first-time transmission/reception and a received signal obtained through second-time transmission/reception is eliminated through amplitude detection by the wave detectors 11 and 12, so that only amplitude information remains. This prevents cancellation between the signals due to a phase difference, and thus even in the case where a subject moves at a high speed, the deterioration of an image quality can be prevented.

Furthermore, according to this embodiment, compared with Embodiment 1, the number of constituent components of the apparatus can be reduced.

The same effect can be obtained also in the case where the memory 3 is located downstream of the wave detector 11.

As described in the foregoing discussion, according to the present invention, when performing synthetic aperture scanning, phases of received signals, each obtained every time transmission/reception is performed, are detected and matched with each other, thereby preventing canceling-out of the signals due to a phase difference caused when a subject moves, and thus an image of an excellent quality can be obtained.

## Claims

1. An ultrasonic diagnostic apparatus using synthetic aperture scanning in which one beam is synthesized through a plurality of times of transmission/reception, comprising:
a transmitting circuit that transmits driving pulses a plurality of times;
a plurality of arrayed transducer-elements, each of which emits an ultrasonic beam from an aperture according to the driving pulses, receives the ultrasonic beam reflected in a subject by means of the aperture, and outputs a received signal;
switches that selectively output a plurality of signals from among the received signals outputted from the arrayed transducer-elements;
a beam synthesizer that performs beam formation based on the signals selected by the switches;
a memory that temporarily stores each of signals outputted from the beam synthesizer as a result of the plurality of times of transmission/reception; and
an adder that adds together the signals in the memory that correspond respectively to the plurality of times of transmission/reception,
wherein the apparatus further comprises:
a unit for detecting a phase difference between the signals obtained through the plurality of times of transmission/reception; and
a delay unit that outputs to the adder the signals obtained through the plurality of times of transmission/reception while matching phases of the signals with each other.

2. The ultrasonic diagnostic apparatus according to claim 1,
wherein the plurality of times of transmission/reception is two times.

3. The ultrasonic diagnostic apparatus according to claim 1,
wherein the unit for detecting a phase difference comprises:
a plurality of zero crossing detectors, each of which detects a timing at which an amplitude of each of the signals that correspond respectively to the plurality of times of transmission/reception shifts from a positive polarity to a negative polarity or vice versa to cross zero; and
a unit for calculating a delay amount with respect to the signals obtained through the plurality of times of transmission/reception based on the timing detected by each of the plurality of zero crossing detectors.

4. An ultrasonic diagnostic apparatus using synthetic aperture scanning in which one beam is synthesized through a plurality of times of transmission/reception, comprising:
a transmitting circuit that transmits driving pulses a plurality of times;
a plurality of arrayed transducer-elements, each of which emits an ultrasonic beam from an aperture according to the driving pulses, receives the ultrasonic beam reflected in a subject by means of the aperture, and outputs a received signal;
switches that selectively output a plurality of signals from among the received signals outputted from the arrayed transducer-elements;
a beam synthesizer that performs beam formation based on the signals selected by the switches;
a memory that temporarily stores each of signals outputted from the beam synthesizer as a result of the plurality of times of transmission/reception; and
an adder that adds together the signals in the memory that correspond respectively to the plurality of times of transmission/reception,
wherein the apparatus further comprises a plurality of wave detectors that perform amplitude detection of the signals obtained through the plurality of times of transmission/reception and output the signals to the adder.

5. The ultrasonic diagnostic apparatus according to claim 4,
wherein the plurality of times of transmission/reception is two times.
